Europäisches Patentamt

European Patent Office

Office européen des brevets

(19)

(11) Publication number: **0 169 684**

**A2**

(12) # EUROPEAN PATENT APPLICATION

(21) Application number: **85304907.0**

(22) Date of filing: **10.07.85**

(51) Int. Cl.⁴: **A 61 K 31/195**
**A 61 K 31/78, A 61 K 33/00**

(30) Priority: **27.07.84 GB 8419245**

(43) Date of publication of application:
**29.01.86 Bulletin 86/5**

(84) Designated Contracting States:
**BE CH DE FR GB IT LI LU NL SE**

(71) Applicant: **RECKITT AND COLMAN PRODUCTS LIMITED**
**P.O. Box 26 1-17, Burlington Lane**
**London W4 2RW(GB)**

(72) Inventor: **Dettmar, Peter William**
**Willow House Main Street**
**Welwick North Humbersida(GB)**

(74) Representative: **Allard, Susan Joyce et al,**
**BOULT, WADE & TENNANT 27 Furnival street**
**London EC4A 1PQ(GB)**

(54) **Pharmaceutical compositions.**

(57) Pharmaceutical compositions comprising mixtures of proglumide and polyacrylic acid or their sodium salts in a specified range of ratios have been found to exhibit synergistic effects in an in vivo test model for anti-ulcer or mucosal-protecting agents. Pharmaceutical compositions comprising mixtures of proglumide and polyacrylic acid or their sodium salts in the range of ratios are described for use in the treatment of gastritis or of gastro-duodenal ulcers.

EP 0 169 684 A2

Croydon Printing Company Ltd.

# PHARMACEUTICAL COMPOSITIONS

This invention relates to pharmaceutical compositions and in particular to compositions for the treatment of gastritis and gastro-duodenal ulcers.

Proglumide (DL-4-benzamido-N,N-dipropylglutaramic acid) has been shown to be an effective drug in the management and prevention of gastroduodenal disorders. The compound has been reported to inhibit gastric acid secretion by antagonising gastrin receptors and it has been suggested that the anti-ulcer activity of this agent was attributable not only to its antisecretory action but also to an additional protective effect. Studies have also demonstrated that proglumide increases the thickness of the gastric mucous gel layer and thus intensifies the protective action of the underlying mucosa.

Sodium polyacrylate has been suggested for use in the treatment of peptic ulcers. British Patent No. 1435630 describes a solid antipeptic ulcer composition comprising sodium polyacrylate having an intrinsic viscosity of 0.3 or more and a pharmaceutically inert solid carrier. British Patent No. 1538352 describes an improved composition which comprises granules of polyacrylic alkali metal salt coated with a water-insoluble but water permeable coating agent. Suitable polyacrylic alkali metal salts are stated to include sodium polyacrylate of molecular weight 3,000,000 to 8,000,000. The only specific sodium polyacrylate mentioned is one of molecular weight about 3,400,000.

We have carried out investigations into the mucosal-protective properties of proglumide and various acid polymers of natural and synthetic origin. We have surprisingly discovered that there is a synergistic effect when proglumide and polyacrylic acid or their sodium salts are mixed in certain proportions.

According to this invention there is provided a pharmaceutical composition comprising proglumide or its sodium salt and polyacrylic acid or its sodium salt in a weight ratio of from 1:3 to 3:1, and preferably from 1:1 to 1:3.

The polyacrylic acid may be linear or cross-linked. Examples of commercial grades of linear polyacrylic acids or their sodium salts are Carbopol 907 (free acid form B.F. Goodrich) and Aronvis (sodium salt Nihon Junyaku). Examples of commercial grades of cross-linked sodium polyacrylates are Rheogic 252L, Rheogic 250 H (Nihon Junyaku), and Hostacerin PN73 (Hoescht U.K. Ltd.) A preferred cross-linked polyacrylic acid is one of the carbomer range (free acid form).

In a preferred aspect of the invention there is provided a pharmaceutical composition comprising proglumide or its sodium salt and carbomer or its sodium salt in a weight ratio of from 1:3 to 3:1, and preferably in a ratio of from 1:1 to 1:3.

Carbomer is described in the British Pharmacopeia and the United States National Formulary as being a synthetic high molecular weight cross-linked polymer of acrylic acid containing 56 to 68% of carboxylic acid groups. The British Pharmacopeia specifies cross-linking with allylsucrose. Carbomer is used in the form of neutralised gel as a suspending agent in pharmaceutical preparations for internal and external uses. U.S. Patent No. 2909462 describes the use as a bulk laxative of a colloidally water-soluble polymer of acrylic acid cross-linked with from about 0.75% to 2.0% of polyallyl sucrose.

Examples of suitable commercial grades of carbomer are those sold by B. F. Goodrich under the Registered Trade Marks Carbopol 910, 934, 934P, 940 and 941. Other examples are those sold by Nihon Junyaku as Junlon PW110, Junlon PW150 and Junlon PW111, and Acrisint 400 (Sigma, Italy). In the compositions of the present invention the preferred material is carbomer 934P, having a molecular weight of approximately 3,000,000, a commercial grade being Carbopol 934P. (See USAN and the USP dictionary of drug names, USAN 1984 page 89).

The compositions of the invention are for oral administration and may be in the form of aqueous compositions having a pH of between 3 and 9.

The invention also includes the use of proglumide or its sodium salt and polyacrylic acid or its sodium salt in a weight ratio of from 1:3 to 3:1, and preferably in a ratio of 1:1 to 1:3 in the treatment of gastritis or gastro-duodenal ulcers.

In the treatment of gastritis or gastro-duodenal ulcers the normal dosage of proglumide or its sodium salt will be in the range 300 to 50 mg and that of polyacrylic acid or its sodium salt in the range 300 to 50 mg provided that the weight ratio of proglumide or its sodium salt to sodium polyacrylic acid or its sodium salt falls within the range of 1:3 to 3:1 and preferably in the range 1:1 to 1:3.

Because of the synergistic effect between the proglumide or its sodium salt and polyacrylic acid or its sodium salt the present compositions afford the possibility of lower doses of proglumide or its sodium salt being used with a resultant reduction in side effects.

The compositions may also include an antacid. Suitable materials include sodium bicarbonate, calcium carbonate, aluminium hydroxide and mixtures thereof. Use of these materials, in particular sodium bicarbonate, also results in a reduction in the viscosity of the liquid compositions, thereby providing some degree of viscosity control in the design of readily pourable liquid preparations.

With aqueous compositions, which are susceptible to contamination and subsequent deterioration by microorganisms it is preferable to include a preservative. A suitable system is a combination of methyl and propyl-p-hydroxy benzoates (methyl paraben or propyl paraben) or their sodium salts.

The pharmaceutical compositions of the present invention may also include one or more of a colouring, sweetening or flavouring agent.

The invention is illustrated by the following Examples.

Examples 1 to 6

Liquid preparations were prepared having the following formulations:

| Example No.           |    | 1     | 2     | 3     | 4     | 5     | 6      |
|-----------------------|----|-------|-------|-------|-------|-------|--------|
| Carbopol 934P         |    | 0.2   | 0.4   | 1.0   | 1.0   | 0.6   | 0.2 g  |
| Proglumide            |    | 0.2   | 0.4   | 1.0   | 1.0   | 0.2   | 0.6 g  |
| Sodium bicarbonate    |    | -     | -     | 1.0   | 1.0   | 0.8   | - g    |
| Calcium carbonate     |    | -     | -     | -     | 1.0   | -     | - g    |
| Methyl paraben, sodium|    | 0.149 | 0.149 | 0.149 | 0.149 | 0.149 | 0.149g |
| Propyl paraben, sodium|    | 0.022 | 0.022 | 0.022 | 0.022 | 0.022 | 0.022g |
| Sodium hydroxide      |    | qs    | qs    | qs    | qs    | qs    | qs     |
| Water                 | to | 100   | 100   | 100   | 100   | 100   | 100 ml |
| pH                    |    | 8.4   | 8.3   | 8.2   | 8.3   | 8.0   | 8.6    |

The carbopol and proglumide were dispersed, with agitation, in about 90 ml water, and aqueous sodium hydroxide added to adjust the pH to about 8. The remaining ingredients were added, followed by additional water to make up to a volume of 100 ml.

Example 7

Carbopol 934P sodium salt was prepared by dispersing 1kg Carbopol 934P in a solution of 400g sodium hydroxide in 3.6 kg anhydrous methanol. The salt was collected by filtration, dried and comminuted.

A blend of 500g Carbopol 934P sodium salt with 500g proglumide sodium and 500g calcium carbonate was mixed, in a planetary mixer, with 1.0L of a 10% w/v solution of polyvinyl pyrrolidone (povidone K30) in isopropanol, and the wet mass passed through an oscillating granulator fitted with a 750μm screen. The granules were dried and rescreened through a 750μm sieve.

320mg Units of these granules, each containing 100mg Carbopol 934P sodium, 100mg proglumide sodium and 100mg

- 6 -                    0169684

calcium carbonate were filled into size 1 hard gelatine capsules.

<u>Examples 8 to 10</u>

Liquid preparations were prepared having the following formulations:

| Example No. | | 8 | 9 | 10 |
|---|---|---|---|---|
| Carbopol 934P | | 0.6 | 0.4 | 0.4 g |
| Proglumide | | 0.2 | 0.4 | 1.2 g |
| Methyl paraben | | 0.13 | 0.13 | 0.13g |
| Propyl paraben | | 0.02 | 0.02 | 0.02g |
| Sodium hydroxide | | qs | qs | qs |
| Water | to | 100 | 100 | 100ml |
| pH | | 4.1 | 4.1 | 4.2 |

The Carbopol, proglumide and parabens were dispersed, with agitation, in about 90 ml water, and aqueous sodium hydroxide added to adjust the pH to about 4. Additional water was then added to make up to a volume of 100 ml.

<u>Example 11</u>

A blend of 500g Carbopol 934P with 500g proglumide and 390g lactose was mixed, in a planetary mixer, with 1.1L of a 10% w/v solution of polyvinyl pyrrolidone (povidone K30) in isopropanol, and the wet mass passed through an oscillating granulator fitted with a 750μm screen. The granules were dried and rescreened through a 750μm sieve.

300 mg Units of these granules, each containing 100 mg Carbopol 934P and 100 mg proglumide were filled into size 1 hard gelatine capsules.

The pharmaceutical properties of the compositions of the invention have been evaluated in two <u>in vivo</u> rat models.

The anti-ulcer or mucosal-protecting properties were determined in the ethanol-induced gastric necrosis test by a method based on that of Robert A., Nezamis J.E., Lancaster C. and Hanchar A.J., Gastroenterology 77, 433, (1979).

In the test method male Sprague-Dawley rats (150-170g) were housed singly and fasted for 18 hours and deprived of water for 4 hours prior to the treatment. Drug or drug vehicle was administered orally (n = 10 per group) in a dose volume of 5 ml/kg. Thirty minutes later the rats were dosed orally with 80% ethanol (in a dose volume of 5 ml/kg). One hour later the rats were killed by cervical dislocation. The abdomen was opened immediately and the stomach exposed. The stomach was tied off at the base of the oesophagus and then removed with 4 to 5 cms of duodenum attached. The gastric contents were flushed out twice with water, then the stomach was inflated using 70% (Industrial Methylated Spirits) and stored in 70% IMS prior to examination. The stomach was opened along the greater curvature and the mucosa was examined by an observer who was unaware of the treatment given. The lesions were measured in millimeters in a systematic manner, their length recorded, and the total lesion length per stomach was determined. The code was broken after all the stomachs had been examined and the severity of the lesion damage was expressed as the mean total lesion length (± SEM) per group of rats. Statistical analysis of the date was performed using student's 't' test for unpaired data. A pretreatment effect was considered significant if the p value was less than 0.05. The control vehicle treated group mean was compared to that of the drug treated group and the percentage protection from the lesion damage caused by ethanol was determined.

The ability of the compositions to bind (adhere) to the rat gastric mucosa was determined by a method based on that of Green A.P., Lander J.E. and Turner D.H., J. Pharm. Pharmacol. 33, 348, (1981). The method employs a cationic dye, alcian blue, that binds to acidic mucopolysaccharides present in gastric mucus. In vitro, this dye gives a positive reaction with polysaccharides e.g. k-carrageanan, alginate, carboxymethyl cellulose and xanthan, and polyacrylates e.g. carbomer.

In the test method male Sprague-Dawley rats (130-150g) were housed singly and fasted overnight for 18 hours before treatment. Following treatment (administered orally in a dose volume of 5 ml/kg) the rats (n=10 per group) were left for a further 60 minutes before being killed by cervical dislocation. The abdomen was opened immediately, the stomach was dissected out, freed of any connective tissue and opened along the greater curvature. The stomachs were gently washed under lightly running water and placed into 10ml of ice-cold 0.25M sucrose solution. The stomachs in the sucrose solution were then weighed on an electronic balance (Sartorius 1212 MP) and the approximate wet weight of each stomach was determined. The stomachs were removed from the sucrose solution and lightly shaken with forceps to remove excess sucrose. The washed stomachs were then incubated in 10ml of freshly prepared Alcian Blue 8GX (Aldrich Chemical) dye solution (1 mg/ml), in 0.15M sucrose buffered with 0.05M sodium acetate that had been adjusted to pH 5.8 with HCl, for two hours at room temperature with an occasional shake. The stomachs (now blue) were washed for 10 minutes with 10ml 0.25M sucrose solution (2x), lightly shaken with forceps to remove excess sucrose and placed in 15ml of 0.5M magnesium chloride solution for a further two hours at room temperature, shaken occasionally, and removed. The blue magnesium chloride solution was shaken for ∿30 seconds with ∿3ml diethyl ether (2x). The optical density of the aqueous layer was measured using disposable cuvettes (4ml capacity, 1cm light path) on a Cecil 595 dual beam spectrophotometer at 605nm. The blank used to compare all the samples (Reference cuvette) was magnesium chloride solution. The results are expressed in optical density units per g tissue weight. Statistical analysis of the data was performed using student's 't' test for unpaired data. A pretreatment effect was considered significant if the p value was less than 0.05. The percentage difference between the control and test groups was also determined.

Tables 1 to 8 present test data for compositions obtained by the two above mentioned in vivo procedures. In Tables 1 to 5 the compositions contained both the polyacrylic acid and the proglumide in the form of their sodium salts whilst in Tables 6 to 8 the free acids were employed.

Table 1 presents test data for compositions having pH 8.0 containing Carbopol 934P (sodium salt) in the rat ethanol-necrosis test.

## Table 1

| Treatment (dose %) | % | |
|---|---|---|
| Carbopol 934P Na | Protection | p values |
| 0.2 | 2.8 | NS |
| 0.4 | 2.9 | NS |
| 0.5 | 25.8 | NS |
| 0.6 | 16.7 | NS |
| 1.0 | 39.1 | <0.02 |
| 2.0 | 34.5 | <0.05 |

Table 2 presents test data for compositions having pH 8.0 containing Carbopol 934P (sodium salt) in the rat gastric binding test. Table 3 shows the time course of binding with a 1% concentration of Carbopol 934P (sodium salt, pH 8.0) in this test.

## Table 2

| Treatment (dose %) | % Increase | |
|---|---|---|
| Carbopol 934P Na | in binding | p values |
| 0.1 | 11.5 | NS |
| 0.2 | 18.8 | <0.05 |
| 0.5 | 75.2 | <0.001 |
| 1.0 | 118.9 | <0.001 |

## Table 3

| Time post | % Increase | |
|---|---|---|
| dose (h) | in binding | p values |
| 0.5 | 109.5 | <0.001 |
| 1 | 118.9 | <0.001 |
| 2 | 58.1 | <0.001 |
| 3 | 65.0 | <0.001 |
| 4 | 36.2 | <0.01 |

## Table 3 (cont.)

| Time post dose (h) | % Increase in binding | p values |
|---|---|---|
| 5 | 36.3 | <0.01 |
| 6 | 6.0 | NS |

From Table 1 it can be seen that Carbopol 934P (sodium salt) possessed only weak mucosal protective activity (39.1% protection, p<0.02 at a 1% dose level) but at this dose level is readily bound to the rat gastric mucosal surface (see Table 2). From Table 3 it can be seen that at a dose level of 1% the binding persisted for upto 5 hours.

Table 4 presents test data for compositions having pH 8.0 containing proglumide (sodium salt) in the rat ethanol-necrosis test.

## Table 4

| Treatment (dose %) Proglumide Na | % Protection | p values |
|---|---|---|
| 0.2 | 10.5 | NS |
| 0.4 | 27.5 | NS |
| 0.6 | 38.0 | <0.05 |
| 2.0 | 79.3 | <0.001 |

From Table 4 it can be seen that proglumide (sodium salt) possessed good mucosal protective activity at the 2% dose level.

Table 5 presents test data for compositions having pH 7.0 to 8.5 containing varying amounts of Carbopol 934P (sodium salt) and proglumide (sodium salt) in the rat ethanol-necrosis test.

## Table 5

| Treatment (dose %) | | % | |
| Carbopol 934P Na + Proglumide Na | | Protection | p value |
|---|---|---|---|
| 0.2 | 0.2 | 50.5 | <0.001 |
| 0.2 | 0.4 | 55.8 | <0.05 |
| 0.2 | 0.6 | 64.8 | <0.02 |
| 0.4 | 0.2 | 46.5 | <0.01 |
| 0.4 | 0.4 | 66.5 | <0.01 |
| 0.4 | 0.6 | 67.9 | <0.001 |
| 0.6 | 0.2 | 62.3 | <0.01 |
| 0.6 | 0.4 | 51.2 | <0.001 |
| 0.6 | 0.6 | 83.0 | <0.01 |

From Table 5 it can be seen that when inactive doses of Carbopol 934P (sodium salt 0.2, 0.4) were combined with an inactive dose of proglumide (sodium salt 0.2) significant protection (50.5%, 46.5%) was produced i.e. synergism was exhibited.

Table 6 presents test data for compositions having pH 3.5 to 3.7 containing Carbopol 934P (free acid) in the rat gastric binding test.

## Table 6

| Treatment (dose %) | % Increase | |
| Carbopol 934P | in binding | p values |
|---|---|---|
| 0.1 | 40.6 | <0.001 |
| 0.2 | 36.3 | <0.001 |
| 0.6 | 51.4 | <0.001 |
| 1.0 | 68.0 | <0.001 |
| 2.0 | 65.2 | <0.001 |

- 13 -                                    0169684

From Table 6 it can be clearly seen that Carbopol 934P is capable of binding (adhering) significantly to the rat gastric mucosal surface when administered as the acid (pH 3.5 to 3.7).

Table 7 presents test data for compositions having pH 3.8 to 4.7 containing proglumide (free acid) in the rat ethanol-necrosis test.

### Table 7

| Treatment (dose %) Proglumide | % Protection | p values |
|---|---|---|
| 0.2 | 9.2 | NS |
| 0.4 | 12.9 | NS |
| 0.6 | 58.6 | <0.01 |
| 2.0 | 64.5 | <0.01 |

From Table 7 it can be seen that proglumide possessed moderate mucosal protection activity at the 0.6% and 2.0% dose levels and further demonstrated that there is no difference in mucosal protective activity between proglumide and its sodium salt.

Table 8 presents test data for compositions having pH 3.0 to 4.2 containing varying amounts of Carbopol 934P and proglumide in the rat ethanol necrosis test.

### Table 8

| Treatment (Dose %) Carbopol 934P | + Proglumide | % Protection | p value |
|---|---|---|---|
| 1.0 | - | 23.0 | NS |
| - | 0.2 | 9.2 | NS |
| 0.2 | 0.2 | 48.7 | <0.02 |
| 0.6 | 0.2 | 69.3 | <0.001 |

From Table 8 it can be seen that when inactive doses of Carbopol 934P were combined with an inactive dose of proglumide (0.2) significant protection (48.7%, 69.3%) was produced.

- 15 -                                    0169684

Claims

1.    A pharmaceutical composition comprising proglumide or its sodium salt and polyacrylic acid or its sodium salt in a weight ratio of 1:3 to 3:1.

2.    A pharmaceutical composition comprising proglumide or its sodium salt and polyacrylic acid or its sodium salt in a weight ratio of 1:1 to 1:3.

3.    A pharmaceutical composition as claimed in Claim 1 or Claim 2 wherein the polyacrylic acid (or its sodium salt) is carbomer (or its sodium salt).

4.    A pharmaceutical composition as claimed in Claim 3 wherein the carbomer is carbomer 934P having a molecular weight of about 3,000,000.

5.    A pharmaceutical composition as claimed in any one of the preceding claims which further includes an antacid.

6.    A pharmaceutical composition as claimed in Claim 5 wherein the antacid is sodium bicarbonate, calcium carbonate or aluminium hydroxide, or mixtures thereof.

7.    A pharmaceutical composition as claimed in any one of the preceding claims in the form of an aqueous composition having a pH of between 3 and 9.

8.    A pharmaceutical composition as claimed in any one of the preceding claims for use in the treatment of gastritis and gastro-duodenal ulcers.